(19) Europäisches Patentamt
European Patent Office
Office européen des brevets

(11) Publication number: 0 067 342
A1

(12) EUROPEAN PATENT APPLICATION

(21) Application number: 82104696.8

(22) Date of filing: 28.05.82

(51) Int. Cl.[3]: C 07 C 43/225
C 07 C 41/22, C 07 B 9/00

(30) Priority: 04.06.81 US 270426

(43) Date of publication of application: 22.12.82 Bulletin 82/51

(84) Designated Contracting States: CH DE FR GB LI

(71) Applicant: HOECHST-ROUSSEL PHARMACEUTICALS INCORPORATED
Route 202-206 North
Somerville New Jersey 08876(US)

(72) Inventor: Shutske, Gregory Michael
22 Runyon Avenue
Somerset New Jersey 08873(US)

(72) Inventor: Lee, Thomas Bing Kin, Dr.
5 Latourette Road
Whitehouse Station New Jersey 08889(US)

(72) Inventor: Jobin, Gregory Michael
201 North Bridge Street
Somerville New Jersey 08876(US)

(74) Representative: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) **Process for the preparation of 2-chloro-1,3-dimethoxybenzene.**

(57) An improved process for the preparation of 2-chloro-1,3-dimethoxybenzene involving the conversion of 1,3-dimethoxybenzene to [2,6-dimethoxyphenyl]-lithium and the reaction of this compound with a chlorinating agent is disclosed. 2-Chloro-1,3-dimethoxybenzene is useful as starting material for the synthesis of pharmacologically valuable 1,2-benzisoxazoloxy acetic acids.

EP 0 067 342 A1

COMPLETE DOCUMENT

Croydon Printing Company Ltd.

The present invention relates to a novel, efficient and relatively inexpensive process for the preparation of 2-chloro-1,3-dimethoxybenzene. More particularly, the present invention relates to a short, technically simple process for the preparation of 2-chloro-1,3-dimethoxybenzene involving lithiating 1,3-dimethoxybenzene to [2,6-dimethoxyphenyl]lithium and contacting the lithio derivative with a chlorinating agent.

The commercial feasibility of processes for the preparation of pharmacologically valuable compounds depends, for the most part, upon the availability and cost of the starting material or materials thereof. For example, the commercial feasibility of the process described in published European Patent Application No. 0 002 666 for the preparation of certain diuretic, uricosuric and antihypertensive 1,2-benzisoxazoloxyacetic acids and derivatives thereof depends, in part, upon the availability and cost of 2-chloro-1,3-dimethoxybenzene, see the following reaction scheme according to the cited EP patent application:

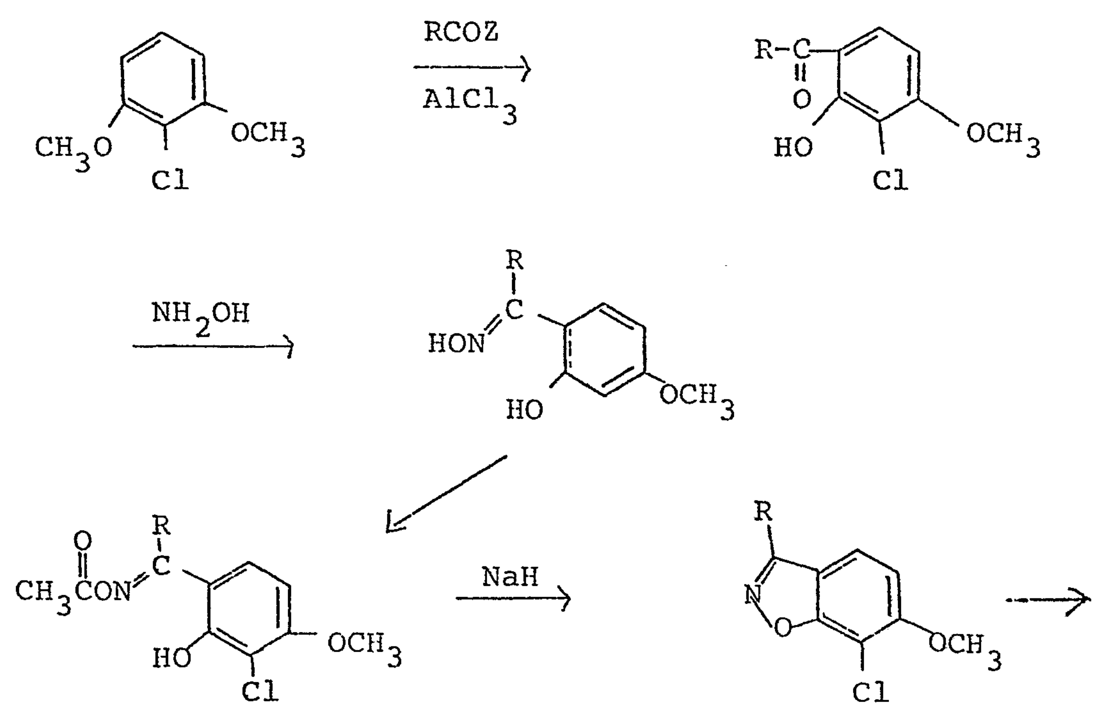

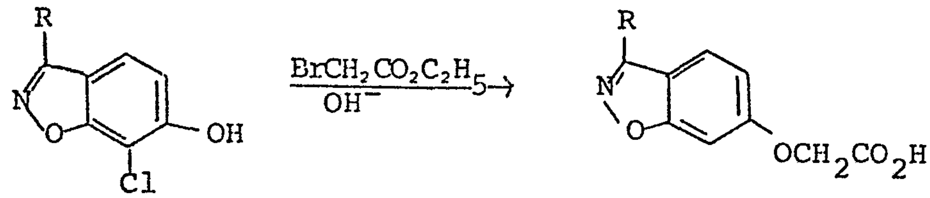


wherein R and Z are as defined in EP Patent Appl. No. 000 26 66.

A process for the preparation of 2-chloro-1,3-dimethoxybenzene was disclosed by N. Schamp in Bull. Chem. Soc. Belges, 73, 35 (1964); Chem. Abs., 60, 7945h (1964) and P. Kovacic and M.E. Kurz in J. Org. Chem., 31, 2459 (1966). This process, however, involves three steps from commercially available cyclohexane-1,3-dione, namely, the chlorination of cyclohexane-1,3-dione followed by dehydrochlorination of the resulting 2,2-dichlorocyclohexane-1,3-dione to 2-chloro-1,3-dihydroxybenzene, as described by N. Schamp, and methylation thereof to 2-chloro-1,3-dimethoxybenzene, as described by P. Kovacic and M.E. Kurz, and thus would apparently furnish the desired chloro compound with difficulty and in relatively low yield, even if the individual steps proceed in relatively high yield, see the following reaction scheme II:

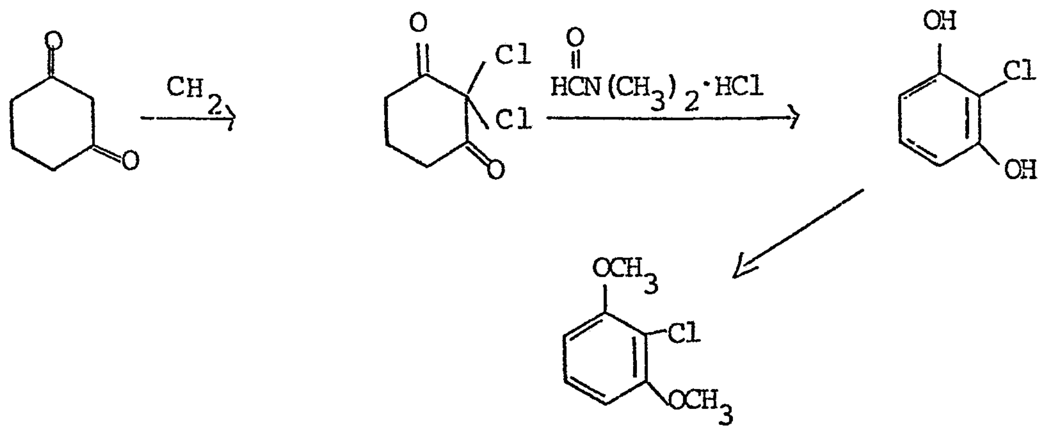

The economics of a process for the preparation of 2-chloro-1,3-dimethoxybenzene would be substantially improved and the availability of this compound for the preparation of pharmacologically important 1,2-benzisoxazoloxyacetic acids would be materially increased if a shorter, technically simpler and more efficient method than that described in the aforementioned references were available. The present invention involves such a short, technically simple and apparently more efficient and less costly process for the preparation of 2-chloro-1,3-dimethoxy-benzene, which comprises the reaction stepts according to the following reaction scheme III:

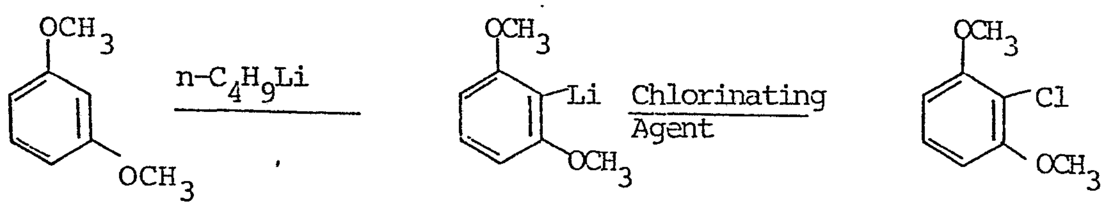


1 2 3

As used throughout the specification and appended claims, the terms "polychloroalkane" refers to an alkane, i.e., a straight or branched chain saturated hydrocarbon, having the empirical formula $C_nH_{2n+2}$ wherein n is 1 to 6 such as methane, ethane, propane, butane, 2-methylbutane, 3-methylpentane and the like, in which the hydrogen atoms have been replaced by chlorine atoms, such as tetrachloromethane, hexachloroethane, octachloropropane, decachlorobutane, 1,1,1,2,3,3,4,4,4-nonachloro-2-trichloromethylbutane,1,1,1,2,2,4,5,5,6,6,6-undecachloro-3-trichloromethylpentane and the like. The term "polychloroalkanone" refers to a polychloroalkane having 3 to 6 carbon atoms in which a dichloromethylene moiety is replaced by a carbonyl function such as hexachloropropan-2-one, octachlorobutan-2-one,1,1,1,3,4,4,4-heptachloro-3-trichloromethylbutan-2-one,1,1,1,3,4,4,5,5,5-

nonachloro-3-trichloromethylpentan-2-one and the like.

In the process of the present invention for the preparation of 2-chloro-1,3-dimethoxybenzene as illustrated in Reaction Scheme III, readily available 1,3-dimethoxybenzene 1 is converted to the 2-lithio derivative, /‾2,6-dimethoxyphenyl_7-lithium 2 by conventional methods well known in the art. Typically, 1,3-dimethoxybenzene dissolved in a suitable solvent, such as diethyl ether or 1,2-dimethoxyethane, is treated with a slight excess of n-butlyllithium and the reaction mixture is either allowed to stand at ambient temperature for, for example, about one to 16 hours, or heated under reflux for about the same time period to complete the conversion. See, for example, K-H. Boltze, et al., Ann. Chem., 709, 63 (1967). Generally without isolation, the lithio derivation 2 is transformed to 2-chloro-1,3-dimethoxybenzene by treatment with a chlorinating agent. Among chlorinating agents, there may be mentioned chlorine, N-chloroimides such as N-chlorosuccinimide, N-chlorophthalimide and the like and polychloroalkanes having 1 to 6 carbon atoms and polychloroalkanones having 3 to 6 carbon atoms, such as those hereinbefore defined. Of the N-chloroimides, N-chlorosuccinimide is preferred. Of the polychloroalkanes, tetrachloromethane and hexachloroethane are preferred. Of the polychloroalkanones, hexachloropropan-2-one is preferred. Hexachloroethane is most preferred.

The transformation of the lithio derivative 2 to the desired chlorobenzene 3 is suitably performed in an inert solvent. Suitable inert solvents include ethereal solvents, such as, for example diethyl ether, tetrahydrofuran, dioxane, 1,2-dimethoxyethane, dimethoxyethoxyethane and the like. The preferred solvent is diethyl ether.

The relative molar amounts of lithio derivative 2, as defined by the initial molar amount of 1,3-dimethoxybenzene 3, and chlorinating agents are not narrowly

critical and excess chlorinating agent may be employed. Generally, small molar excess of the chlorinating agent in the order of about 5 to 10 % is preferred to promote the conversion of 2 to 3 without causing purification problems. When chlorine is employed as the chlorinating agent, however, it is desirable to limit the molar excess of chlorine to about 10 % to avoid possible complicating side-reactions.

While the reaction temperature is also not narrowly critical, it is preferred to perform the conversion of 2 to 3 at a temperature at which the reaction proceeds to completion at a convenient rate. When and N-chloroimides are employed as the chlorinating agent, the reaction proceeds at a convenient rate at a temperature within the range of about 35°C to 75°C, a most convenient rate being attained at a temperature of about 55°C. When polychloroalkanes are utilized, the reaction proceeds at a convenient rate at a temperature within the range of about -80°C to about 35°C, a most convenient rate being attained at a reaction temperature of about 25°C. When polychloroalkanones are chosen as the chlorinating agent, the conversion proceeds at a convenient rate at a temperature within the range of about -75°C to about 25°C, a most convenient rate being attained at a temperature of about 0°C. In the case of chlorine, i.e., when gaseous chlorine is used as the chlorinating agent, a convenient reaction rate is attained at a temperature within the range of about 30°C to 50°C, a most convenient rate being attained at a temperature at about 50°C.

2-Chloro-1,3-dimethoxybenzene is useful as a starting material for the preparation of 7-chloro-1,2-benzisoxazoloxyacetic acids and derivatives thereof, which show diuretic, uricosuric and antihypertensive properties, as described in published EP Patent Application No. 000 26 66.

The following example is for illustrative purposes only and is not to be construed as limiting the invention described herein in any way whatsoever.

Example

Preparation of 2-Chloro-1,3-dimethoxybenzene

a) N-Chlorosuccinimide Method

To a solution of 1,3-dimethoxybenzene (13.8 g) in 1,2-dimethoxyethane (100 ml) is added 2.6M n-butyllithium (40 ml). After 40 minutes, N-chlorosuccinimide (13.5 g) is added and the reaction temperature is maintained below 55°C by means of an ice-bath. After an additional 45 minutes, the reaction mixture is poured into water and extracted with ether. Evaporation of the organic extract affords an oil which is chromatographed on silica gel (125 g), eluting with 20 % ether-hexane. Evaporation of the eluents affords an oil which crystallized on standing. Recrystallization from hexane gives 2-chloro-1,3-dimethoxybenzene, mp 62°-64°C.

ANALYSIS:

| | | | |
|---|---|---|---|
| Calculated for $C_8H_9ClO_2$: | 55.66 % C | 5.25 %H | 20.54 % Cl |
| Found: | 55.75 % C | 5.20 %H | 20.24 % Cl |

(b) Chlorine Method

Gaseous chlorine is condensed in a receptacle immersed in a dry ice-acetone bath until 2.5 ml is collected. The bath is removed and the chlorine is allowed to evaporate into a solution of /¯2,6-dimethoxyphenyl_7lithium in 1,2-dimethoxyethane, prepared by adding 2.6M n-butyllithium (10 ml) to 1,3-dimethoxybenzene (6.9 g) dissolved in dimethoxyethane (50 ml) maintained at less than 50°C by means of an ice-bath and permitting the resulting solution to stand for 40 minutes. After one hour, the reaction mixture is poured into water and extracted with ether. The ethereal extracts are dried, filtered and evaporated to afford an oil. A comparison of the proton magnetic resonance spectrum of the oil with that of authentic 2-chloro-1,3-dimethoxybenzene prepared by Method (a) indicated that the reaction product contained 62 % of the 2-chloro compound.

(c) Hexachloroethane Method

To a solution of 1,3-dimethoxybenzene (13.8 g) in dry

ether (50 ml, freshly distilled from sodium benzophenone ketyl) is added dropwise 2.4M n-butyllithium (50 ml) at 5°C over five minutes. After the addition is complete, the mixture is heated under reflux for two and one-half hours and then cooled to -2°C. To the cooled mixture is added dropwise a solution of hexachloroethane (30.9 g) in dry ether (80 ml) over 23 minutes, during which time the reaction temperature increased to 14°C. After this addition is complete, the reaction mixture is allowed to warm to room temperature and is stirred at room temperature overnight. The reaction mixture is cooled to 5°C and water (50 ml) is added. The layers are separated and the ether layer is washed with water (50 ml). The aqueous phase is extracted with ether (50 ml) and the combined organic extracts are dried over anhydrous magnesium sulfate, filtered and evaporated. Recrystallization of the residue from hexane (60 ml) yields 2-chloro-1,3-dimethoxybenzene (12.3 g, 71.5 %), mp 71°-73°C (reported mp 69°C-71°C, P. Kovacic and M.E. Kurtz, ibid. page 2465).

Variations of the basic process involving reaction times, reaction temperatures and reaction solvents did not materially alter the overall results of the method.

(d) Carbon Tetrachloride Method

To a solution of 1,3-dimethoxybenzene (13.8 g) in dry ether (50 ml, freshly distilled over sodium benzophenone ketyl) is added dropwise 2.2M n-butyllithium over a period of 15 minutes, maintaining the reaction temperature at 31°C. After the addition is complete, the reaction mixture is heated under reflux for two hours and stirred at room temperature overnight. The reaction mixture is cooled to -75° to -78°C and a solution of carbon tetrachloride (10.6 ml) in dry ether (20 ml) is added dropwise over a period of 1 hour and 15 minutes, maintaining the reaction temperature at less than -65°C.

After the addition is complete, the reaction mixture is stirred at about -70°C for one hour and allowed to warm to room temperature over a two-hour period. The reaction mixture is cooled to 0°C and ice water (50 ml) and 3N hydrochloric acid (75 ml) are added. Toluene (150 ml) is added. The mixture is filtered and the layers are separated. The aqueous layer is extracted with toluene (60 ml) and the combined organic fractions are washed with 5 % sodium chloride solution (100 ml), dried over anhydrous potassium carbonate, filtered and concentrated to afford an oil (20 g). Gas chromatographic analysis of the oil indicated that it contained predominantly 2-chloro-1,3-dimethoxybenzene together with minimum amounts of starting material, 1,3-dimethoxybenzene.

Ether (60 ml) and hexane (20 ml) are added to the oil. The resulting solid is collected. Gas chromatographic analysis of the solid indicates that it also contains predominantly 2-chloro-1,3-dimethoxybenzene together with lesser amounts of the starting 1,3-dimethoxybenzene.

(e) Hexachloroacetone Method

To a solution of 1,3-dimethoxybenzene (13.8 g) in dry ether (50 ml, freshly distilled from sodium benzophenone ketyl) is added dropwise 2.4M n-butyllithium (50 ml) at 5°C over five minutes. After the addition is complete, the mixture is heated under reflux for two and one-half hours and then cooled to -65°C. To the cooled mixture is added dropwise a solution of hexachloroacetone (19.8 ml) in dry ether (50 ml) over one hour. The reaction mixture is allowed to warm to 0°C and after one hour, water (50 ml) is added and the combined aqueous phases are extracted with ether. The combined ether fractions are dried over anhydrous magnesium sulfate and filtered. The filtrate is washed with 15 % sodium sulfate solution, dried over anhydrous magnesium sulfate,

filtered and evaporated. Trituration of the residue with hexane (41 ml) affords 2-chloro-1,3-dimethoxybenzene (8.6 g, 50 %) as a gum.

The gas-liquid chromatogram of the gum showed that it contained predominantly the desired 2-chloro compound.

1. A process for the preparation of 2-chloro-1,3-dimethoxybenzene of the formula 3

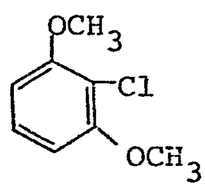

3

which comprises converting 1,3-dimethoxybenzene of the formula 1

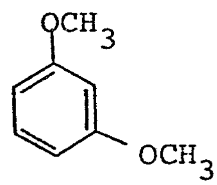

1

by conventional methods to the 2-lithio derivative of the formula 2

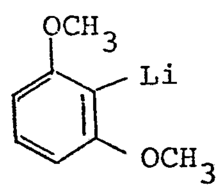

2

and contacting this compound with a chlorinating agent.

2. The process of claim 1 wherein the chlorinating agent is chlorine.

3. The process of claim 1 wherein the chlorinating agent is an N-chloroimide.

4. The process of claim 1 wherein the N-chloroimide is N-chlorosuccinimide.

5. The process of claim 1 wherein the chlorinating agent is a polychloroalkane having 1 to 6 carbon atoms.

6. The process of claim 5 wherein the polychloroalkane is carbon tetrachloride.

7. The process of claim 5 wherein the polychloroalkane is hexachloroethane.

8. The process of claim 1 wherein the chlorinating agent is a polychloroalkanone having 3 to 6 carbon atoms.

9. The process of claim 8 wherein the polychloroalkanone is hexachloracetone.

10. The process of claim 1 wherein the process is performed in the presence of a solvent.

11. The process of claim 10 wherein the solvent is an ethereal solvent.

12. The process of claim 11 wherein the ethereal solvent is diethyl ether.

0067342

European Patent Office

# EUROPEAN SEARCH REPORT

Application number

EP 82 10 4696.8

| Category | Citation of document with indication, where appropriate, of relevant passages | Relevant to claim | CLASSIFICATION OF THE APPLICATION (Int. Cl. 3) |
|---|---|---|---|
| Y | Chemical Abstracts vol. 74, no. 23<br>7 June 1971<br>Columbus, Ohio, USA<br>O. COLLERA ZUNIGA et al. "Synthesis of aromatic derivatives with alkyl substituents via organolithium intermediates"<br>page 422, column 2, abstract no. 125032w<br>& Bol. Inst. Quim. Univ. Nac. Auton. Mex. vol. 22, 1970, pages 152 to 176<br>-- | 1 | C 07 C 43/225<br>C 07 C 41/22<br>C 07 B 9/00 |
| D | JUSTUS LIEBIGS "Annalen der Chemie"<br>Vol. 709,<br>1967, VERLAG CHEMIE, Weinheim/Bergstr.<br>K.H. BOLTZE et al. "Über 2-substituierte Resorcindimethyläther"<br>pages 63 to 69 | | **TECHNICAL FIELDS SEARCHED (Int.Cl. 3)**<br>C 07 B 9/00<br>C 07 C 41/22<br>C 07 C 43/225 |
| Y | * page 65 * | 1 | |
| A | * page 69 * | 10-12 | |

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 23-08-1982 | KNAACK |

EPO Form 1503.1 06.78